# EUROPEAN PATENT SPECIFICATION

(11) **EP 3 247 370 B1**
(45) Date of publication and mention of the grant of the patent: **02.06.2021**
(21) Application number: 16703820.7
(22) Date of filing: 19.01.2016
(51) Int. Cl.: A61K 36/15, A61P 29/00

(54) **WATER SOLUBLE CONIFEROUS RESIN FOR TREATING AND PREVENTING STERILE INFLAMMATION**
WASSERLÖSLICHES NADELBAUMHARZ ZUR BEHANDLUNG UND VORBEUGUNG STERILER ENTZÜNDUNGEN
RÉSINE DE CONIFÈRE SOLUBLE À L'EAU POUR LE TRAITEMENT ET LA PRÉVENTION DE L'INFLAMMATION STÉRILE

(30) Priority: 20.01.2015 FI 20155045
(43) Date of publication of application: 29.11.2017
(73) Proprietor: Repolar Pharmaceuticals Oy, 02270 Espoo (FI)
(72) Inventor: SIPPONEN, Pentti, 02270 Espoo (FI)
(74) Representative: Kolster Oy Ab
(86) International application number: PCT/FI2016/050021
(87) International publication number: WO 2016/116667

(56) References cited:
- DE-A1- 4 311 048
- JP-A- 2003 160 433
- RO-A2- 126 901
- RO-A2- 126 902
- RO-A2- 126 903
- RO-A2- 126 905
- XIAN-WEN YANG ET AL: "Anti-inflammatory and anti-tumour effects of Abies georgei extracts", JOURNAL OF PHARMACY AND PHARMACOLOGY, vol. 60, no. 7, 1 July 2008 (2008-07-01), pages 937-941, XP055259343, LONDON; GB ISSN: 0022-3573, DOI: 10.1211/jpp.60.7.0017
- JANNE J. JOKINEN ET AL: "Refined Spruce Resin to Treat Chronic Wounds: Rebirth of an Old Folkloristic Therapy", ADVANCES IN WOUND CARE, 20 January 2014 (2014-01-20), XP055259364, ISSN: 2162-1918, DOI: 10.1089/wound.2013.0492
- S. P. CLARK ET AL: "Pine Oil Effects on Chemical and Thermal Injury in Mice and Cultured Mouse Dorsal Root Ganglion Neurons", PHYTOTHERAPY RESEARCH., vol. 28, no. 2, 18 April 2013 (2013-04-18) , pages 252-260, XP055259383, GB ISSN: 0951-418X, DOI: 10.1002/ptr.4991
- DATABASE GNPD [Online] MINTEL; October 2008 (2008-10), anonymous: "Resin Salve", XP002755655, Database accession no. 991111
- BROZA SARIC-KUNDALIC: "Traditional Medicine in the Pristine Village of Prokosko Lake on Vranica Mountain, Bosnia and Herzegovina", SCIENTIA PHARMACEUTICA, vol. 78, no. 2, 1 January 2010 (2010-01-01), pages 275-290, XP055176800, ISSN: 0036-8709, DOI: 10.3797/scipharm.1003-06
- DATABASE GNPD [Online] MINTEL; June 2003 (2003-06), anonymous: "Petro-Carbo Salve", XP002755656, Database accession no. 10140696
- Anonymous: "Amazon.com: Customer Reviews: J.R. Watkins Apothecary Petro-carbo medicated first aid salve 4.37 oz", , 12 October 2014 (2014-10-12), pages 1-2, XP055259484, Retrieved from the Internet: URL:http://www.amazon.com/Watkins-Apotheca ry-Petro-carbo-medicated-first/product-rev iews/B0000X070K/ref=cm_cr_arp_d_viewopt_ky wd?ie=UTF8&showViewpoints=1&sortBy=helpful &pageNumber=1&filterByKeyword=psoriasis [retrieved on 2016-03-18]
- A. Sipponen ET AL: "Beneficial effect of resin salve in treatment of severe pressure ulcers: a prospective, randomized and controlled multicentre trial", British Journal of Dermatology, vol. 158, no. 5, 1 May 2008 (2008-05-01), pages 1055-1062, XP055635526, UK ISSN: 0007-0963, DOI: 10.1111/j.1365-2133.2008.08461.x
- SIPPONEN ARNO ET AL: "Resin-salve from Norway spruce - a potential method to treat infected chronic skin ulcers?", DRUG METABOLISM LETTERS,, vol. 1, no. 2, 1 January 2007 (2007-01-01) , pages 143-145, XP008135464, ISSN: 1872-3128

## Description

### Field of the invention

The present invention relates to an anti-inflammatory agent comprising water soluble coniferous resin acids for use in treating or preventing sterile inflammation, wherein the agent is provided as an aqueous solution of coniferous resin or coniferous rosin. The invention is also directed to a pharmaceutical formulation comprising said agent for that use.

### Background of the invention

It is known that coniferous resin and rosin are antimicrobial; i.e., they are microbicidal against a large range of micro-organisms including bacteria and fungi (1-5). For this purpose, natural coniferous resins and rosins have been used even from ancient times, and in traditional medicine, as salve in treatment of infected skin wounds, foreign body lesions, and skin burns (6, 12, 13, 14, 15, 16, 17).

Natural coniferous resin is traditionally collected from trunks of coniferous trees ("callus resin"). Another source of resin raw materials is rosin, which is a fraction that is obtained from distillations of industrial tall oil, which is a byproduct in paper industry. "Callus" resin and industrial rosin can be purified and dissolved into carrier solvents (like alcohol) for manufacturing of salves and medicines with known and patented techniques (7). Coniferous resin contains i.a. resin acids, which are enriched in coniferous rosin. Components of purified coniferous resin, rosin or resin acids can be further mixed with various solvents, or mixed even with water as emulsions, for example, by using sugar alcohols (e.g. glycerol) as co-solvent and "carrier" (8). Such aqueous coniferous resin acids containing compositions are useful e.g. in treating infections and inflammation associated with infections in humans and animals.

Anti-inflammatory and anti-tumour effects of Abies georgei acetate extracts have been disclosed (9). A salve prepared from pure resin of Norway spruce has been shown to have anti-inflammatory potential in wound healing (10). Resin based pine oil has shown to have effects on dermal inflammations in mouse models (11). A topical composition comprising coniferous resin acids for use in treating skin disorders like lichen and psoriasis is disclosed (18). The same document discloses also a topical composition in a form of salve, wherein coniferous resin acids are mixed with tallow (1:2). Picea abies ethanol extract was shown to inhibit MMP3 activity and compositions used for a anti-aging effects on the skin were disclosed (19).

Infection is caused by foreign organisms that invade a host's body and often cause a disease in the host. Infection elicits an immune response in the host's body including formation of antibodies and activation of phagocytes. Infection is normally associated with inflammation. However, there is also another type of inflammation that is not associated with infection. This is called sterile inflammation.

Sterile inflammatory tissue reactions play a pathogenetic role in human and veterinary medicine in a large group of diseases. These reactions are normally targeted to regulate cell renewal, tissue repair and maturation, vascularization, homeostasis, among the others. Even though the renewal, repair and normal physiological cell and tissue functions are dependent on a manifold regulatory chains and actions of cytokines, imbalanced and failed expressions of these pro-inflammatory cytokines result in undesirable side effects. Such side effects are, for example, pain, tissue edema and redness, among others. Abnormal reactions may cause abnormal repair, failures in maturation and regrowth of the tissues, as the case is, for example, in psoriasis, cicatrix keloids, dermatitis herpetiformis, and in pemphigoid or lichenoid skin diseases, or in healing of skin wounds. In skin, abnormal or overexpression of the cytokines emphasizes formation of scars, redness, edema, keloids and scurf, and may result in prolongation in healing of the lesions. In rheumatoid arthritis, arthrosis and synovial diseases cytokines have a pathogenetic role in causing pain, swelling or thickness in synovial and mesenchymal tissues. Sterile inflammatory processes also play a role in diseases and irritations of eye, ears, nasal or oral cavities, anal and perianal area of the gut or women's vulva (e.g., vulvodynia) or vagina.

In medical practice, corticosteroids are the only main medicines used in treatment of the above mentioned skin and rheumatoid diseases in which failures, overexpression or imbalances in expression of cytokines and growth factors may occur. These medicines are commonly used also in treatment of various inflammatory diseases in the gut, like ulcerative colitis, Crohn's disease or apthoid lesions in gastrointestinal cavities, and in a manifold other diseases, including also inflammatory diseases of the eye e.g., dry eyes, ear or oral cavity.

Corticosteroids are the common tools that are used to control sterile inflammatory tissue reactions. Even though the corticosteroids (like dexamethasone) have excellent anti-inflammatory effects, they also have drawbacks in compliance and in appearance of various side effects. Corticosteroids cannot be used for long time periods because of the side effects due to their influences on general metabolism. They interact with cortisone metabolisms and will increase the risk of microbial infections. The present invention provides means for eliminating or reducing such risks and limitations in the treatment and prevention of sterile inflammation. The invention provides an alternative for the use of corticosteroids. This alternative is further advantageous in that it simultaneously provides an antimicrobial effect.

### Brief description of the invention

The invention is an application of natural water soluble coniferous resins and coniferous rosins as medical agents in drugs, solution and salves with the purpose to reduce the expression of the pro-inflammatory cytokines in biological tissues, i.e. to be applied as anti-inflammatory agents. It should be noted that the anti-inflammatory effect is other than the anti-microbial effect, and indicates the sterile reaction of biological tissues and cells. Abnormal or overexpression of the pro-inflammatory cytokines and growth factors is a pathogenetic background phenomenon in a large variety of inflammatory diseases in both humans and animals, especially in mammals. The present invention is a new application whereby natural water soluble coniferous resins or their components, e.g., coniferous resin, rosin, purified coniferous resin acids, or products containing these compounds, can be applied as anti-inflammatory medicines or agents.

The present invention provides an anti-inflammatory agent comprising water soluble coniferous resin acids for use in treating or preventing sterile inflammation in human or animal, wherein the agent is provided as an aqueous solution of coniferous resin or coniferous rosin.

The invention also provides a pharmaceutical formulation comprising an anti-inflammatory agent comprising water soluble coniferous resin acids, wherein the agent is provided as an aqueous solution of coniferous resin or coniferous rosin for use in treating or preventing sterile inflammation in human or animal.

Embodiments not falling under the scope of the appended claims, viz. the agent not being provided as an aqueous solution of coniferous resin or coniferous rosin, are to be considered merely as examples suitable for understanding the invention.

### Brief description of the drawings

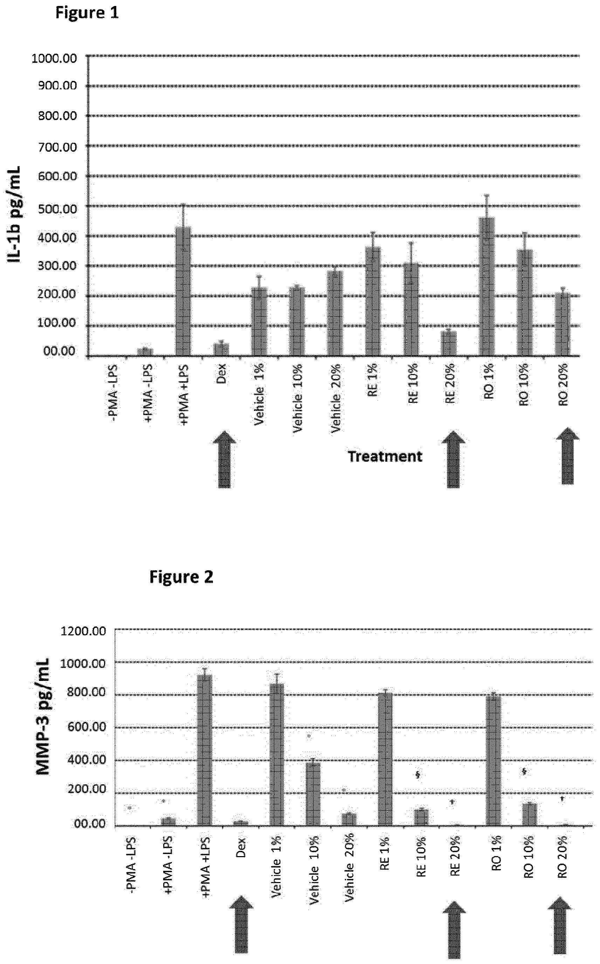

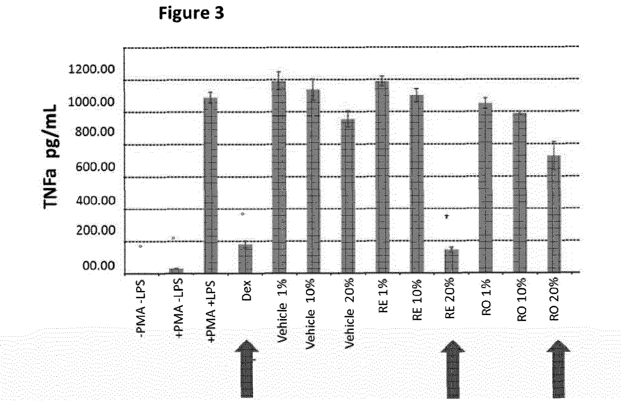

Figures 1-3 present the results from *in vitro* experiments which demonstrate the ability of coniferous resin (RE) and coniferous rosin (RO) to decrease cytokine expressions from a human monocyte cell line.

### Detailed description of the invention

The present invention is based on the application of water soluble coniferous resin or coniferous rosin, and the medical products manufactured from these compounds, as anti-inflammatory agents in human or veterinary medicine. The invention specifically relates to reducing and down-regulating the expression of the pro-inflammatory cytokines and growth factors in human and animal tissues; i.e., applying agent comprising resin acid in medical indications that correspond to those of the corticosteroids at present. The agents can be water-bound, i.e., coniferous resins are prepared in water or saline formulations.

"Sterile inflammation" refers to tissue reactions which are mediated by so-called pro-inflammatory cytokines and/or growth factors, and in which microbes do not play an initial role or initial cause of the inflammation, and which are associated with swelling and redness without the presence of active microbe infection, and in which corticosteroids conventionally are the medicine of choice. Typically, the sterile inflammation is a condition that responds to treatment with a corticosteroid. Especially the sterile inflammation is selected from the group consisting of sterile inflammation in the skin, mucosa, joint, tendon, eye, ear, nasal or oral cavity, and gastrointestinal tract.

In one embodiment, the sterile inflammation is selected from the group consisting of psoriasis; cicatrix keloids; scars; seborrheic, herpetiformic, pemphigoid or lichenoid skin diseases; skin itching, scurf or dandruff; rheumatoid diseases like rheumatoid arthritis; arthrosis; tendinitis; synovial diseases including pain, swelling or thickness in synovial and mesenchymal tissues; autoimmune diseases and irritations of the eye, ears, nasal or oral cavities, dry eye syndrome, irritation of anal and perianal area and of women's vulva or vagina; vulvodynia; autoimmune and inflammatory diseases in the gastrointestinal tract; ulcerative colitis; Crohn's disease; apthoid lesions in the gastrointestinal tract mucosa; anal fissures or haemorrhoids; proctitis; and inflammatory lesions in anal glands. The anti-inflammatory agent comprising resin acids is not only useful in the treatment of said diseases but also in preventing them. In one embodiment it is administered a subject that is suffering from or susceptible to a condition that responds to treatment with a corticosteroid.

The anti-inflammatory agent used is a composition comprising at least one coniferous resin acid, usually a mixture of coniferous resin acids, and it typically reduces the expression of proinflammatory cytokines and/or growth factors, wherein the cytokines preferably are selected from the group consisting of IL-1b, MMP-3, Cox-2 and TNFα.

The anti-inflammatory agent comprising coniferous resin acid is conveniently provided as water soluble coniferous resin or coniferous rosin. The dispersion is usually an aqueous dispersion/solution as described in (8) or an oil-in-water dispersion/emulsion, or a water-in-oil dispersion/emulsion as described below. The anti-inflammatory agent can also be further purified from the resin, rosin or dispersion thereof.

"Coniferous resin" refers to natural callus resins conveniently collected from trunks of coniferous trees. It is a mixture of various chemical compounds, composed of i.a. p-coumaric acid, resin acids, lignans, fatty acids and volatile terpenic compounds.

"Coniferous rosin" refers to the resin acid fraction obtained from distillation of tall oil obtained from kraft pulping of coniferous trees. It consists mainly of coniferous resin acids. Typically it contains about 95% coniferous resin acids and about 3% fatty acids.

"Coniferous resin acid" refers to organic acids found in resin and/or rosin, such as hydroxylated derivates of hydroxydehydroabietic acid. In one embodiment the coniferous resin acids are selected from the group consisting of dehydroabietic acid, 7β-hydroxydehydroabietic acid, 7α-hydroxydehydroabietic acid, 15-hydroxydehydroabietic acid, 7β,15-dihydroxydehydroabietic acid, 7α,15-dihydroxydehydroabietic acid, 18-hydroxydehydroabietic acid, further hydroxylated derivates of dehydroabietic acid and a mixture thereof.

The anti-inflammatory agent comprising resin acids can be applied in various pharmaceutical formulations, e.g. in the form of an aqueous solution, optionally comprising a pharmaceutically acceptable diluent, carrier and/or excipient. The formulation can be in the form of a salve, cream, ointment, gel, suppositorium, enema, vagitorium, aqueous or oil solution, eye drops, injectable solution or oil, liniment, solid or liquid feed, dietary supplement, chewing gum, dog or cat chew bone, shampoo, liniment, lotion, stick, spray, or solid drug or pill. The pharmaceutical formulation may also comprise other pharmaceutically active agents such as hydrocortisone, cortisone, glucoseamine and dwxpanthenol, and/or conventional excipients and/or functional additives.

The pharmaceutical formulation may be administered orally, intravenously, intra-arterially, subcutaneously, intramuscularly, topically or as an injection or infusion. Salves, creams, suppositories, vagitories, solutions, shampoos or liniments in various formulations are products typically applicable in treatment of skin diseases, anal diseases and gynecological (vaginal) diseases. Aqueous or oily liniments, injectable solutions are usable in medicines targeted to joint cavities, tendons or for intramuscular use. Per oral drugs, solutions, enemas, suppositories or feeds are tools for treatment or prevention of gut diseases. In one embodiment the pharmaceutical formulation is in the form of an injectable solution that is applied for treatment of joint cavities, muscular lesions or tendons in rheumatoid arthritis, arthrosis or tendinitis.

The anti-inflammatory agents and pharmaceutical formulations described can be prepared and manufactured industrially from coniferous resin and coniferous rosin with known techniques.

Tinctures with ethanol or mixtures of coniferous resins, rosins or resin acids with oils or fats (butter, animal fats, etc.) are well known techniques by which the resin or rosin products can be prepared and manufactured. Mixtures of resin with fats or oils can be used in preparation of salves, creams, suppositories, vagitories, etc. Impregnation of these resin products into solid carrier substances (e.g., starch) enables the preparation of solid drugs or capsules. There is for example a resin salve that is used in treatment of infected chronic wounds and burns, and is manufactured as 10% (w/w) resin salve industrially (Abilar® Resin salve), and as 30% (w/w) lacquer (Abicin®) for nail fungal infections, by Repolar Oy, Finland.

Aqueous, water intermixable resin/rosin compositions can be prepared and manufactured by adding water or saline into a mixture that composes coniferous resin/rosin in a dispersing agent like alcohol, and especially glycerin, and, thereafter, by separating the aqueous fraction (the layer of water dispersion) from the mixture. The resulting aqueous fraction is a dispersion that contains water soluble resin acids (8). The resin is typically first dissolved in ethanol, which is then optionally removed to obtain a solid resin product. The resulting resin in ethanol or solid resin is then dissolved in glycerin e.g. 1 part resin in ethanol and 9 parts of glycerin and mixed. Saline is then added to obtain a dispersion having a glycerin:saline ratio of 1:1 (vol/vol), and the precipitate formed is separated to obtain an aqueous solution containing coniferous resin acids. The thus obtained stock-solution may be used as such, or further diluted with saline to a final glycerin concentration of e.g. 5-30%(w/w), preferably 10-20%(w/w). Glycerin and glycerol are used as synonyms. Saline (0.9% NaCl) may be replaced by water. AniVox® (20%) marketed as an antimicrobial ear wash for dogs is an example of such a product that is manufactured as described above, and marketed commercially at present.

An aqueous resin/rosin dispersion can also be prepared by using oils (e.g. organic oils such as vegetable oils like olive oil, palm oil, flaxseed oil or rape-seed oil, or mineral oils) as dispersing agent instead of glycerin, and recovering the aqueous and/or the oil phase. Coniferous resin or rosin is optionally first dissolved into a volatile carrier substance such as ethanol or other organic solvent. The optionally dissolved resin or rosin is mixed with oil in a resin/rosin concentration of 1-50 %, preferably 5-30%, most preferably 10-20% (w/w). The volatile substance is left to evaporate. An aqueous medium such as saline is added to the oil mixture in a selected volume ratio that may vary in the ratio of 1:50 to 50:1, typically it is up to 1:15, preferably it is about 1:1 (vol/vol). The mixture is left to stabilize for 1-12 hours, whereby an upper water-in-oil fraction and a lower oil-in-water fraction are formed, which both have anti-inflammatory activity. The fractions are separated. In one embodiment the oil-in-water fraction is filtered and applied as an aqueous anti-inflammatory solution. In another embodiment the water-in-oil fraction is used.

When the anti-inflammatory agent is used as an aqueous solution, the total concentration of resin acids is typically about 1-1000 mg/L (ppm), usually about 1-500 mg/L, preferably about 1-200 mg/L, e.g. 10-100 mg/L.

The innovation is based on the finding that water soluble coniferous resin acid containing agents show marked anti-inflammatory properties that are comparable even with those of dexamethasone; i.e., they suppress the expression of cytokines from human monocyte cells in a significant manner even in low concentrations (Figs 1-3). The tests and studies indicate that (i) the water soluble parts of both coniferous callus resin and industrial rosin (resin acid fraction of industrial tall oil) reduce the expression of all studied three main cytokines, IL-1b, MMP-3 and TNFα, from human monocyte-macrophage cell line: i.e., they have a strong anti-inflammatory property, and that (ii) this anti-inflammatory influence is as marked as that of dexamethasone; and that (iii) this inhibitory action is received with low concentrations of resin acids; (iv) the anti-inflammatory actions are mediated by both resins (natural "callus" resins collected from trunks of coniferous trees) and rosins ("industrial" distilled rosin fraction obtained from tall oil and enriched in resin acid), indicating that the resin acids are the likely mediators of the anti-inflammatory actions of the resin and rosin solution, which suggests, therefore, further (v) that the products manufactured and prepared from natural resin and rosin have these anti-inflammatory properties.

The marked anti-inflammatory influence was apparent even with a resin or rosin solution, which was diluted to 20% (1:4) with cell culture medium in the *in vitro* experiments, the resin and rosin stock solution being 50% glycerin in saline and containing resin acids ad 100 mg/L (ppm) (Figs 1-3).

When natural coniferous resin or rosin is dissolved into saline or water, a final solution is obtained in which the concentration of water soluble resin acids typically varies between 10 and 500 mg/L, but is mostly between 50 and 150 mg/L (8). Aqueous solutions of this type are biologically effective, as shown in Figs 1-3, and have influences on expression of cytokines in living human cells and tissues. Several case reports indicate that resin products, both in formulations of salve and aqueous solution diminishes the clinical symptoms and scurf in psoriatic skin lesions.

### Example 1

Coniferous callus resin or coniferous rosin was dissolved in ethanol for 3-4 weeks to provide a solution comprising 60% (dry weight) resin or rosin. 40 ml of this ethanol solution was mixed for one day with 200 ml of pure glycerin to provide a mixture. The mixture was diluted with 240 ml of saline (1:1 by volume). A precipitation of insoluble resin acids was formed and removed by filtration. A colloidal dispersion was obtained as a filtrate. The filtrate was allowed to stand overnight at room temperature to evaporate the ethanol from the filtrate. The obtained stable aqueous resin (RE) or rosin (RO) stock solutions comprising 50% glycerin (vol/vol) contain water soluble coniferous resin acids (e.g., hydroxylated derivates of hydroxydehydroabietic acid) in concentrations around 100 ppm (see reference 8). These aqueous solutions were then diluted to 20% (diluted 1 to 4), 10% or 1% test solutions with cell culture medium. The anti-inflammatory effects of the test solutions were investigated.

U937 cells, of a human monocyte cell line (MD Biosciences, Inflammations Discovery Service, Altum Building, Todd Campus, West Of Scotland Science Park, Maryhill Road, Glasgow, G20 0XA, UK; contract MD-2-2-144-1370), United Kingdom, were used. The cells were treated with 200 nM phorbol 12-myristate 13-acetate (PMA) for 72 hours to differentiate them into macrophages. PMA was then removed and the cells incubated with media control, vehicle control (25% glycerin in saline), 0.5 microM dexamethasone or 3 test items: 1%, 10% or 20% (v/v) RE, 1%, 10% or 20% (v/v) RO for 1 hour according to the protocol. After 1 hour the cells were treated with 1 microg/mL of LPS and unstimulated cells were treated with incomplete media of the same volume. After 20 hours of LPS stimulation cell viability was assayed by adding AlamarBlue™ to each well, incubating for 1 hour and measuring the fluorescence, 544nm excitation and 590nm emission. After 24 hours of LPS stimulation the well supernatants were harvested and subsequently analysed for IL-1b, MMP-3 and TNFα levels by Luminex multiplex according to the manufactures' instructions. The effects of dexamethasone (positive control) and 20% RE and RO are emphasized in the Figures with arrows.

In the figures vehicle indicates resin or rosin free saline - glycerin solutions in which glycerin concentration is 25% (vol/vol). LPS (lipopolysaccharide) is the stimulator which was used to activate the synthesis and secretion of cytokines and growth factors in the U937 cells. RE and RO are abbreviations of the aqueous coniferous resin and rosin solutions prepared as described in reference (8).

The results show that coniferous resin and rosin (and resin acids) that dissolve from resins or rosins to water (tissue fluids) have marked anti-inflammatory properties that are comparable even with those of dexamethasone; i.e., they suppress the expression of cytokines from human monocyte cells in significant manner even is low concentrations (Figs 1-3). These aqueous solutions in dilution to 20% (diluted 1 to 4) with cell culture medium significantly reduced the expression of IL-1b, MMP-3 and TNFα in a manner that corresponds to that obtained with dexamethasone. The anti-inflammatory effect is obvious and marked with both RE and RO indicating that the reduction of expression of cytokines and growth factors from human cells is caused by the coniferous resin acids.

### Example 2 (Reference)

Resin in a concentration of 10% (w/w) in a formulation of oil-based salve was spread topically on skin lesions to patients having psoriasis. Several case reports from patients with psoriasis indicated improvement of symptoms with the salve, particularly in psoriasis of small spot types (*Psoriasis guttata* and *Psoriasis pustulosa*)*.*

### Example 3

A stock solution comprising coniferous resin in a mixture of saline with glycerin (1/1; vol/vol) was prepared as described in Example 1 and diluted with saline to provide a dispersion comprising 20% glycerin. An aqueous skin lotion in which the aqueous part of the lotion was said diluted dispersion was applied on the scalp. Rapid improvements of dandruff with this lotion were noted and reported.

### Example 4

A stock solution comprising coniferous resin in a mixture of saline with glycerin (1/1; vol/vol) is prepared as described in Example 1 and diluted with saline to provide a dispersion comprising 20% glycerin. The dispersion is used as an anti-inflammatory ingredient in a formulation for intra-articular injection, this ingredient being the aqueous part of the injection fluid. The injection is given to subjects having rheumatoid arthritis or tendinitis.

### Example 5

In formulation of a suppositorium, coniferous resin was an ingredient in a concentration of 30% (w/w). The effect of the salve to reduce itching and to heal fissures was studied. Prospective observations in 10 consecutive patients with anal fissures indicated similar improvement of the fissures with the resin salve as with a corticosteroid salve.

### Example 6

100 ml of ethanol containing 60 g coniferous resin prepared as described in Example 1 is mixed with 1000 ml of rape-seed oil. Ethanol is left to evaporate and 1000 ml physiological saline is added. The solution is left to stabilize for 1-12 hours to obtain an upper water-in-oil layer, and a lower oil-in-water layer. The oil-in-water fraction is recovered and filtered to obtain an aqueous solution.

Eye drops, wherein the aqueous part of the drop formulation is the obtained oil-in-water fraction diluted to 20% (1:4) with physiological saline are given to patients having dry eye syndrome.

### Example 7

A dog or cat chew bone containing resin mixed with solid matrix substance in a concentration of 5 % (w/w) was given to dogs and cats suffering from gingival irritation and pain. A relieve of pain by the resin was observed.

### Example 8

A stock solution comprising coniferous resin in a mixture of saline with glycerin (1/1; vol/vol) is prepared as described in Example 1 and diluted to provide a dispersion comprising 20% glycerin. A gel formulation, wherein the aqueous part of the formulation is the dispersion is given to reduce irritation and mucosal sensitivity in vagina and vulva.

A salve containing 10% (w/w) coniferous resin was given to patients having vulvodynia. Pain symptoms were observed to be healed in six consecutive patients with severe vulvodynia.

### Example 9 (Reference)

Resin in a formulation of a 10% salve, cream or lotion was used to prevent or treat skin scars and cicatrix. The formulation was spread on a wound scar to reduce the expression of pro-inflammatory cytokines to prevent the overgrowth of the granulation tissue, and to prevent pain. Decrease of the visibility of the scars and cicatrix was observed.

### Example 10

A water soluble and water mixable resin dispersion as an ingredient of enema is used to reduce the expression of tissue cytokines in colon mucosa in autoimmune inflammation and colitis.

### References

1. Rautio M, Sipponen A, Peltola R, Lohi J, Jokinen JJ, Papp A, Carlson P, Sipponen P. Antibacterial effects of home-made resin salve from Norway spruce (Picea abies). APMIS 2007; 115: 335.-40
2. Sipponen A, Laitinen K. Antimicrobial properties of natural coniferous rosin in the European Pharmacopoeia challenges test. APMIS 2011; 119: 720-24
3. Rautio M, Sipponen A, Lohi J, Lounatmaa K, Koukila-Kähkölä P, Laitinen K. In vitro fungistatic effects of natural coniferous resin from Norway spruce (Picea abies). Eur J Clin Micr Inf Dis 2012; 31: 1783-89
4. Sipponen A, Jokinen JJ, Sipponen P, Papp A, Sarna S, Lohi J. Beneficial effect of resin salve in treatment of severe pressure ulcers: a prospective, randomized and controlled clinical trial. Br J Dermatol 2008; 158: 1055-62
5. Sipponen A, Kuokkanen 0, Tiihonen H, Jokinen JJ. Natural coniferous resin salve to treat complicated surgical wounds: pilot clinical trial on healing and costs. Int J Dermatology 2912; 51: 726-32
6. Sipponen A. Coniferous resin salve, ancient and effective treatment for chronic wounds - laboratory and clinical studies. Academic Dissertation, 2013; Helsinki University, Helsinki, Finland. ISBN 978-952-10-8710-3
7. Finnish patent FI 122,095 (Repolar Oy)
8. EP patent publication EP 2,775,838 (Patolab Oy)
9. XIAN-WEN YANG ET AL: "Anti-inflammatory and anti-tumour effects of Abies georgei extracts", JOURNAL OF PHARMACY AND PHARMACOLOGY, vol. 60, no. 7, 1 July 2008 (2008-07-01), pages 937-941
10. JANNE J. JOKINEN ET AL: "Refined Spruce Resin to Treat Chronic Wounds: Rebirth an Old Folkloristic Therapy", ADVANCES IN WOUND CARE, 20 January 2014
11. S. P. CLARK ET AL: "Pine Oil Effects on Chemical and Thermal Injury in Mice and Cultured Mouse Dorsal Root Ganglion Neurons",
12. BROZA SARIC-KUNDALIC: "Traditional Medicine in the Pristine Village of Prokosko Lake on Vranica Mountain, Bosnia and Herzegovina", SCIENTIA PHARMACEUTICA, vol. 78, no. 2, 1 January 2010 (2010-01-01), pages 275-290,
13. DATABASE GNPD [Online] MINTEL; June 2003 (2003-06), anonymous: "Petro-Carbo Salve", Database accession no. 10140696
14. R0126905 A2
15. R0126901 A2
16.R0126902 A2
17. R0126903 A2
18. DE4311048 A1
19. JP2003160433 A

## Claims

1. An anti-inflammatory agent comprising water soluble coniferous resin acids for use in treating or preventing sterile inflammation in human or animal, wherein the agent is provided as an aqueous solution of coniferous resin or coniferous rosin.

2. A pharmaceutical formulation comprising an anti-inflammatory agent comprising water soluble coniferous resin acids, wherein the agent is provided as an aqueous solution of coniferous resin or coniferous rosin for use in treating or preventing sterile inflammation in human or animal.

3. The anti-inflammatory agent for use according to claim 1, or the pharmaceutical formulation for use according to claim 2, wherein the sterile inflammation is a condition that responds to treatment with a corticosteroid.

4. The anti-inflammatory agent for use according to claims 1, or the pharmaceutical formulation for use according to claim 2, wherein the sterile inflammation is selected from the group consisting of sterile inflammation in the skin, mucosa, joint, tendon, eye, ear, nasal or oral cavity, and gastrointestinal tract.

5. The anti-inflammatory agent or the pharmaceutical formulation for use according to claim 4, wherein the sterile inflammation is selected from the group consisting of psoriasis; cicatrix keloids; scars; seborrheic, herpetiformic, pemphigoid or lichenoid skin diseases; skin itching, scurf or dandruff; rheumatoid diseases like rheumatoid arthritis; arthrosis; tendinitis; synovial diseases including pain, swelling or thickness in synovial and mesenchymal tissues; autoimmune diseases and irritations of the eye, ears, nasal or oral cavities, dry eye syndrome, irritation of anal and perianal area and of women's vulva or vagina; vulvodynia; autoimmune and inflammatory diseases in the gastrointestinal tract; ulcerative colitis; Crohn's disease; apthoid lesions in the gastrointestinal tract mucosa; anal fissures or haemorrhoids; proctitis; and inflammatory lesions in anal glands.

6. The anti-inflammatory agent for use according to claim 1, or the pharmaceutical formulation for use according to claim 2, wherein the agent reduces the expression of proinflammatory cytokines or growth factors, selected from the group consisting of IL-1b, MMP-3, and TNFα.

7. The pharmaceutical formulation for use according to claim 2, wherein the formulation also comprises a pharmaceutically acceptable diluent, carrier and/or excipient.

8. The pharmaceutical formulation for use according to claim 7, wherein the formulation is in the form of a salve, cream, ointment, gel, suppositorium, enema, vagitorium, eye drops, injectable solution, liniment, solid or liquid feed, dietary supplement, chewing gum, dog chew bone, shampoo, liniment, lotion, stick, spray, or solid drug or pill.

9. The pharmaceutical formulation for use according to claim 7, wherein the total concentration of coniferous resin acids in the aqueous solution is 1-1000 mg/L, preferably 10-100 mg/L.

10. The pharmaceutical formulation for use according to claim 2, wherein the formulation is in the form of an injectable solution that is applied for treatment of joint cavities, muscular lesions or tendons in rheumatoid arthritis, arthrosis or tendinitis.

11. The pharmaceutical formulation for use according to claim 2, wherein the formulation is administered orally, intravenously, intra-arterially, subcutaneously, intramuscularly, topically or as an injection or infusion.

## Patentansprüche

1. Entzündungshemmendes Mittel, umfassend wasserlösliche Nadelbaumharzsäuren, zur Verwendung beim Behandeln oder Verhindern einer sterilen Entzündung bei einem Menschen oder Tier, wobei das Mittel als eine wässrige Lösung von Nadelbaumharz oder Nadelbaumkolophonium bereitgestellt wird.

2. Pharmazeutische Formulierung, umfassend ein entzündungshemmendes Mittel, umfassend wasserlösliche Nadelbaumharzsäuren, wobei das Mittel als eine wässrige Lösung von Nadelbaumharz oder Nadelbaumkolophonium bereitgestellt wird, zur Verwendung beim Behandeln oder Verhindern einer sterilen Entzündung bei einem Menschen oder Tier.

3. Entzündungshemmendes Mittel zur Verwendung nach Anspruch 1 oder pharmazeutische Formulierung zur Verwendung nach Anspruch 2, wobei die sterile Entzündung ein Leiden ist, das auf eine Behandlung mit einem Kortikosteroid anspricht.

4. Entzündungshemmendes Mittel zur Verwendung nach Anspruch 1 oder pharmazeutische Formulierung zur Verwendung nach Anspruch 2, wobei die sterile Entzündung aus der Gruppe bestehend aus einer sterilen Entzündung in der Haut, einer Schleimhaut, einem Gelenk, einer Sehne, einem Auge, einem Ohr, einer Nasen- oder Mundhöhle und dem Magen-Darm-Trakt ausgewählt ist.

5. Entzündungshemmendes Mittel oder pharmazeutische Formulierung zur Verwendung nach Anspruch 4, wobei die sterile Entzündung aus der Gruppe bestehend aus Psoriasis; Narbenkeloiden; Narben; Seborrhoe-, Herpetiformis-, Pemphigoid- oder Lichenoid-Hautkrankheiten; Hautjucken; Grind oder Schuppen; Rheumakrankheiten, wie rheumatoide Arthritis; Arthrose; Tendinitis; Synovialkrankheiten, einschließlich Schmerz, Anschwellen oder Verdickung in Synovial- und Mesenchymgeweben; Autoimmunkrankheiten und Reizungen der Augen, Ohren, Nasen- oder Mundhöhle, trockenem Auge, Reizung des Anal- oder Perianalbereichs und der Vulva oder Vagina von Frauen; Vulvodynie; Autoimmun- und Entzündungskrankheiten im Magen-Darm-Trakt; Colitis ulcerosa; Morbus Crohn; aphthoiden Läsionen in der Schleimhaut des Magen-Darm-Trakts; Analfissuren oder Hämorrhoiden; Proktitis und entzündlichen Läsionen in den Analdrüsen ausgewählt ist.

6. Entzündungshemmendes Mittel zur Verwendung nach Anspruch 1 oder pharmazeutische Formulierung zur Verwendung nach Anspruch 2, wobei das Mittel die Expression von proinflammatorischen Zytokinen oder Wachstumsfaktoren, die aus der Gruppe bestehend aus IL-1b, MMP-3 und TNFα ausgewählt sind, verringert.

7. Pharmazeutische Formulierung zur Verwendung nach Anspruch 2, wobei die Formulierung außerdem ein pharmazeutisch annehmbares Verdünnungsmittel, einen pharmazeutisch annehmbaren Trägerstoff und/oder einen pharmazeutisch annehmbaren Hilfsstoff umfasst.

8. Pharmazeutische Formulierung zur Verwendung nach Anspruch 7, wobei die Formulierung in der Form von einer Salbe, einer Creme, einem Balsam, einem Gel, einem Suppositorium, einem Einlauf, einem Vagitorium, Augentropfen, einer injizierbaren Lösung, einem Einreibemittel, einem festen oder flüssigen Futtermittel, einem Nahrungsergänzungsmittel, einem Kaugummi, einem Hundekauknochen, einem Shampoo, einem Einreibemittel, einer Lotion, einem Stick, einem Spray oder einem festen Wirkstoff oder einer festen Pille ist.

9. Pharmazeutische Formulierung zur Verwendung nach Anspruch 7, wobei die Gesamtkonzentration von Nadelbaumharzsäuren in der wässrigen Lösung 1-1000 mg/L, vorzugsweise 10-100 mg/L beträgt.

10. Pharmazeutische Formulierung zur Verwendung nach Anspruch 2, wobei die Formulierung in der Form einer injizierbaren Lösung ist, die zur Behandlung von Gelenkhöhlen, Muskelläsionen, oder Sehnen bei rheumatoider Arthritis, Arthrose oder Tendinitis ist.

11. Pharmazeutische Formulierung zur Verwendung nach Anspruch 2, wobei die Formulierung oral, intravenös, intraarteriell, subkutan, intramuskulär, topisch oder als eine Injektion oder Infusion verabreicht wird.

## Revendications

1. Agent anti-inflammatoire comprenant des acides de résine de conifère solubles dans l'eau, pour une utilisation dans le traitement ou la prévention d'une inflammation stérile chez un humain ou un animal, lequel agent se présente sous la forme d'une solution aqueuse de résine de conifère ou de colophane de conifère.

2. Formulation pharmaceutique comprenant un agent anti-inflammatoire comprenant des acides de résine de conifère solubles dans l'eau, dans laquelle l'agent se présente sous la forme d'une solution aqueuse de résine de conifère ou de colophane de conifère, pour une utilisation dans le traitement ou la prévention d'une inflammation stérile chez un humain ou un animal.

3. Agent anti-inflammatoire pour une utilisation selon la revendication 1, ou formulation pharmaceutique pour une utilisation selon la revendication 2, dans lequel l'inflammation stérile est un état qui répond à un traitement par un corticostéroïde.

4. Agent anti-inflammatoire pour une utilisation selon la revendication 1, ou formulation pharmaceutique pour une utilisation selon la revendication 2, dans lequel l'inflammation stérile est choisie dans le groupe constitué par une inflammation stérile de la peau, d'une muqueuse, d'une articulation, d'un tendon, d'un œil, d'une oreille, du nez ou de la cavité orale, et des voies gastro-intestinales.

5. Agent anti-inflammatoire ou formulation pharmaceutique pour une utilisation selon la revendication 4, dans lequel l'inflammation stérile est choisie dans le groupe constitué par un psoriasis ; les chéloïdes cicatriciels ; les cicatrices ; les maladies cutanées séborrhéiques, herpétiformes, pemphigoïdes ou lichénoïdes ; une irritation cutanée, des squames ou des pellicules ; les maladies rhumatoïdes telles que la polyarthrite rhumatoïde ; l'arthrose ; la tendinite ; les maladies synoviales comprenant une douleur, un gonflement ou un épaississement des tissus synoviaux et mésenchymateux ; les maladies auto-immunes et les irritations des yeux, des oreilles, des cavités nasales ou orales, un syndrome de sécheresse oculaire, une irritation de la zone anale et périanale et de la vulve ou du vagin chez les sujets de sexe féminin ; une vulvodynie ; les maladies auto-immunes et inflammatoires des voies gastro-intestinales ; la recto-colite hémorragique ; la maladie de Crohn ; les lésions aphtoïdes de la muqueuse des voies gastro-intestinales ; les fissures anales ou les hémorroïdes ; une proctite ; et les lésions inflammatoires des glandes anales.

6. Agent anti-inflammatoire pour une utilisation selon la revendication 1, ou formulation pharmaceutique pour une utilisation selon la revendication 2, dans lequel l'agent réduit l'expression de facteurs de croissance ou de cytokines pro-inflammatoires, choisis dans le groupe constitué par IL-1b, MMP-3, et TNF-α.

7. Formulation pharmaceutique pour une utilisation selon la revendication 2, laquelle formulation comprend aussi un diluant, véhicule et/ou excipient pharmaceutiquement acceptable.

8. Formulation pharmaceutique pour une utilisation selon la revendication 7, laquelle formulation est sous la forme d'une pommade, d'une crème, d'un onguent, d'un gel, d'un suppositoire, d'un lavement, d'un suppositoire vaginal, de gouttes oculaires, d'une solution injectable, d'un liniment, d'un aliment solide ou liquide, d'un complément alimentaire, d'une gomme à mâcher, d'un os à mâcher pour chien, d'un shampooing, d'un liniment, d'une lotion, d'un bâtonnet, d'un spray, ou d'un médicament ou pilule solide.

9. Formulation pharmaceutique pour une utilisation selon la revendication 7, dans laquelle la concentration totale d'acides de résine de conifère dans la solution aqueuse est de 1 à 1000 mg/l, de préférence de 10 à 100 mg/l.

10. Formulation pharmaceutique pour une utilisation selon la revendication 2, laquelle formulation est sous la forme d'une solution injectable qui est appliquée pour le traitement de cavités articulaires, de lésions musculaires ou de tendons lors d'une polyarthrite rhumatoïde, d'une arthrose ou d'une tendinite.

11. Formulation pharmaceutique pour une utilisation selon la revendication 2, laquelle formulation est administrée par voie orale, intraveineuse, intra-artérielle, sous-cutanée, intramusculaire, topique, ou sous la forme d'une injection ou perfusion.
